# EUROPEAN PATENT SPECIFICATION

(11) **EP 2 737 879 B1**
(45) Date of publication and mention of the grant of the patent: **18.05.2016**
(21) Application number: 13183859.1
(22) Date of filing: 11.09.2013
(51) Int. Cl.: A61F 5/01

(54) **Aid grabbing tool with hand operated gripper that can be pushed aside**
Hilfsgreiferwerkzeug für handbetätigten Greifer, der zur Seite geschoben werden kann
Outil de broutage assisté d'un outil de préhension manuel pouvant être repoussé

(30) Priority: 29.11.2012 DK 201200755
(43) Date of publication of application: 04.06.2014
(73) Proprietor: MANUXA ApS, 6400 Sønderborg (DK)
(72) Inventor: Pedersen, Hans Joergen, 24955 Harrislee (DE); Hansen, Jesper Allan, 6310 Broager (DK)
(74) Representative: Inspicos P/S

(56) References cited:
- WO-A1-2011/137904
- US-A- 4 231 603
- US-A- 5 800 561

## Description

This invention relates to aid devices for assisting people inhibited in their movement of especially the hands and/or wrists, such as people who are physically impaired due to arthritis, injury, or other physiological problems giving reduced strength. This is solved by introducing an aid tool with at least two grippers and means to attach each gripper to a finger of a person, where the grippers are adapted to follow the finger(s) in at least the directions to grab and release an object, and where the grippers are adapted to be positioned at the sides of the fingers of the person. When the aid tool has been moved to grab an object by a person, an activation of the aid tool ensures a unidirectional locking of the grippers, meaning they are locked in their movement in the releasing direction.

### Background of the invention

This invention relates to aid devices for assisting people inhibited in their movement of especially the hands and/or wrists, such as people who are physically impaired due to arthritis, injury, or other physiological problems giving reduced strength.

A substantial number of people suffer from deformations, chronic pain, and other impairments of the hands and wrists, due to injuries or various diseases such as rheumatoid or other forms of arthritis. Such impairments often render it difficult or impossible for the affected people to hold and effectively to use and handle tools, for example such as a pen, pencil, or other writing instrument, scissors, toothbrushes, kitchen utensils (such as knives, forks, stirrers, spatulas, etc.), and the like.

Many such people are able move their arm, and even fingers, but need strength to grip devices and hold them firmly.

The aim of the present invention therefore is to introduce a device, either single-or multi-purpose aid device, which can assist or enable people with physically impaired hands and/or wrists to hold and use various hand-held tools and devices (such as toothbrushes, keys, scissors, tongs, and kitchen utensils) with greater ease, where the invention especially introduces a tool to assist gripping objects firmly, this being especially suited for users able to move their arms, hands and fingers, only having no strength to take a firm grip on objects. Especially it is an aim of the present invention to introduce a device with grippers to be placed between the user's fingers and an object to be held, so that the holding force exerted by the aid tool does not affect the fingers but only increases the pressure between the grippers of the aid tool and the object.

One drawback of prior art aid tools, such as they are illustrated in e.g. US 4,167,044 is the grippers are either positioned 'on top' of the fingers of the user, thus pressing the fingers onto the object to be grabbed, or they are positioned 'inside the hand' of the user, see e.g. US 5,800,561 or not yet published application PCT/DK2011/000044 (WO 2011/137904 A1). Each of these strategies offers drawbacks.

If the grippers are 'on top' of the fingers, when an object is grabbed, the hand/fingers of the user are being fixed and 'squeezed' between the aid tool and the object, which may be a discomfort.

If the grippers are 'inside the hand' of the user, all feeling of the contact of the user to the object will be lost, since the contact purely are 'mechanical' as the contact will be of the aid tool to the object.

The user of such aid devices or tools may sometimes wish to use the tool, and then shortly after not, this instead of having to constantly attach and detach the tool, it would be advantageous if at least the gripping part of the device may be easily disengaged and removed from the immediate operation range of the users hand, only to easily be grabbed by the users hand again when needed. The present invention introduces an aid tool of the same kind as disclosed in PCT/DK2011/000044, but where the drawback as mentioned above is solved.

### Summary of the system

This is solved by introducing an aid tool of the same kind as disclosed PCT/DK2011/000044 with most of the disclosed features, such as comprising at least two grippers and means to attach each gripper to a finger of a person, where the grippers are adapted to follow the individual finger in at least the directions to grab and release an object. When the aid tool has been moved to grab an object by a person, an activation of the aid tool ensures a unidirectional locking of the grippers, meaning they are locked in their movement in the releasing direction.

In this manner the users may themselves move the hand and thereby the aid tool to the object and grab it. The grippers follow the fingers in at least the directions to grab and release an object and a hold force, in the following a hold, on the object is subsequently established to ensure a firm grip even though the user has no significant strength. This hold is formed by the aid tool being adapted to tighten the grip on the object a little more than the strength of the grip at the time of activation.

In the present invention the drawbacks are solved by introducing the aid tool according to claim 1.

The movable section is connected to the mounting means through joints or a bendable section allowing relative movement of the movable section to the mounting means.

To ensure the tool will not just 'swing' back to inhibit the operation range of the users hand, the movable section locks in the pushed aside position through build in resilience in the joints or bendable section. It may in one embodiment be that the movable section is locked in the pushed aside position unlocks again when a switch is activated, such as when the grippers is pushed or squeezed at some predetermined force.

To identify when the predetermined force is reached the aid tool may includes a force sensor measuring the force whereby the grippers is pushed or squeezed, or alternatively the force whereby the grippers is pushed or squeezed is measured measuring the strain gauge of one or more of the grippers.

In intelligent versions the movable section automatically moves to the pushed aside position when no activation has been made for a given time.

In one embodiment, to overcome discomfort of the user not feeling the object, then at least one gripper may be adapted to be positioned between the fingers of the user, where the term 'between' is to be understood in the sense it is essentially positioned at the side of user fingers seen in an orthogonal direction relative to the direction of grapping

### Figures

- Fig. 1: A simple illustration of the aid tool of the basic principle gripper of the present invention.
- Fig. 2: An illustration one basic embodiment of a setup of the aid tool of the present invention.
- Figs. 3A and B: Simple illustration of the basic of the present invention where the aid tool includes a movable section able to be pushed aside.
- Fig. 4: One embodiment of how the movable section may be locked into one position.
- Fig. 5: A top view of a simple illustration of the aid tool disclosing one embodiment of the contact platform according to the present invention.
- Fig. 6: A top view of a simple illustration of the aid tool disclosing a second embodiment of the contact platform according to the present invention.

### Detailed description of the invention

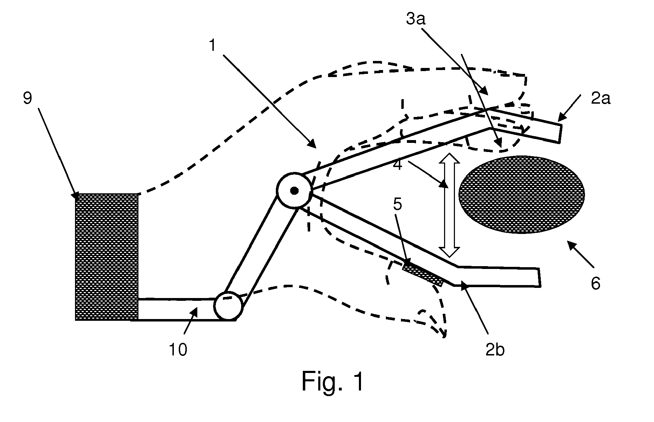

Fig. 1 shows schematically the concept of an aid tool (1) according to the present invention, where the illustration is a side view of one embodiment setup having at least one gripper (2a) positioned at the side of the fingers (3a) of the user, where 'side' is defined as being in the direction orthogonal to the direction of grapping and releasing as illustrated by the arrow (4). At least one gripper (2b) may be adapted such that the associated finger may press onto the gripper (2b), where the associated finger in the illustrated embodiment is the thumb (3b), and it may as in the illustrated embodiment comprise a switch (5)

The grippers (2) are able to move with the fingers at in at least the directions to grab and release (4) an object (6).

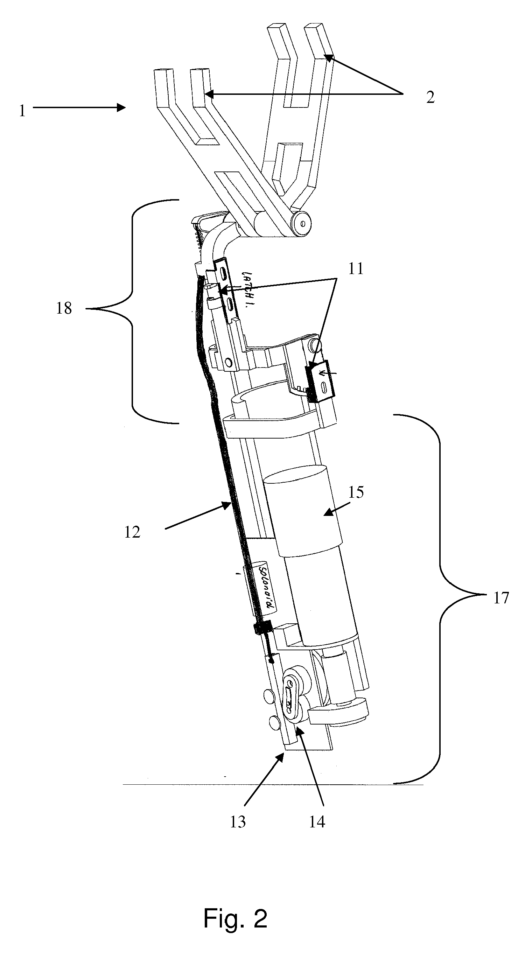

When the aid tool (1) is placed in connection with an object (4), Fig. 2, the user grabs on the object with the grippers (2) and the fingers.

In the illustrated embodiment, at least one of the grippers (2b) has an activation switch (5) positioned such that when the associated finger (in the illustration the thumb (3b)) press the activation switch (5) with a pre-defined force, a gripper interlocking mechanism is activated (not illustrated) to lock preferably all the grippers (2) in their present position in an unidirectional manner meaning they are locked in their movement in the releasing direction but may be free to move in the grapping direction.

The aid tool (1) is further adapted to form a hold on an object (6), this hold being formed by tightening the grip on the object (6) a little more than the strength of the grip at the time of activation. This further gives a need that the grippers (2) do no press onto the fingers (3) of the user, or this extra tightening grip might be pain full.

Fig. 2 illustrates one non-limiting embodiment of the aid tool (1) according to the present invention.

The figure shows the aid tool (1) with the grippers (2) being coupled (18) to the driving unit (17), where the driving unit (17) comprises all the means to drive and control the grippers (2), and is the part attached to the mounting means (9). The mounting means (9) is the means that in any known manner is fixed securely to the user, such as at the wrist or arm. The coupling (18) preferably comprise two flexible joints (11) where they respectively gives an up-and-down and a side-to-side movement, or rotation, of the aid tool (1) in relation to the driving unit (17). These flexible joints (11) however are adapted to lock in their rotation when the aid tool (1) is activated, thus 'freezing' the relative positions at least substantially into their relative positions at the time of activation.

At least one of the grippers (2) is driven by the wire (12) pulling the grippers (2), and where the wire (12) is connected to a rack (13), where the rack (13) again is being operated by a pinion-system (14) in a manner where the teeth of the rack and connected pinion is shaped such that the connected pinion may prevent the rack from movement in the direction to loosen the grip of the grippers (2) when the system as activated, but does not prevent it to move in the direction to fasten the grip (this being another example of the principle of unidirectional tightening explained earlier).

A craftsman will recognise how to make the teeth of rack (13) and pinion (14) to achieve this, or any other manner where a similar effect may be achieved for example by having the pinion (14) locking in rotation in one but not the opposite direction, by motor control, by actuator control etc. Further the illustrated embodiment comprises an electric motor (15) to drive e.g. the pinion system (14). The present invention is not limited to the exact manner this is achieved, but to the important aspect, that even though the grippers (2) have been locked by activation of the system, this is only to be understood as locking in the loosening direction, not in the tightening direction of the grippers (2). The persons using the aid tools (1) may themselves fasten the grip by tightening the grippers (2) with their own fingers, the system preventing the grip to be loosened again even though the person loosen the grip of the fingers, at least until the system is deactivated again.

In one preferred embodiment the system operates such that after activation the grip of the grippers (2) the hold will be established by tightening the grip slightly more than it was at the time of activation, where this is driven by the electric motor (15). How much the grippers (2) further will tighten the grip may depend on several factors, like being pre-defined, depending on some activation of the user of the aid tool (2) optionally operated by the second hand and/or depending on a strain gauge of one or more of the grippers (2) adapted to measure the tightening force, where e.g. one or more of these may be used by and algorithm to calculate the further tightening grip.
Fig. 2 only illustrates one possible embodiment of the aid tool (1), alternative actuating systems to the electromotor (15) may be introduced just as alternatives to the wires (12), rack (13) and pinion (14) system etc.

The grippers (2) may be operated by drivers, such as for example electrical, electrostatic, magnetic, pneumatic, hydraulic, mechanical or string means, such as by for example actuators of any kind as known in the art. These drivers may be controlled by pressing the activation switches (5) at the fingers at a predetermined force.

In one alternative embodiment the hold is established in that at least some of the grippers (2) comprise a contact section to the object (6) being an inflatable or expandable contact section adapted to form protrusions above the surface of the gripper when the aid tool (1) is activated, these protrusions forming at least part of the contact surface to the object (6).

When the aid tool (1) is activated by pressing the activation switches (5), the inflatable or expandable contact sections inflate, much as a balloon, or expands, to a predetermined pressure or expansion level and reach out of the grippers (2) until they get into the desired degree of contact with the object (6). A combination of the unidirectional locked grippers (2), the inflatable or expandable contact sections (squeezing onto the object (6)) and their high friction surfaces ensures a firm grip onto the object.

The main aspect of forming the hold is that after activation the grippers (2) form a slightly tighter grab than at the time of activation. The grippers (2) may then comprise contact sections being elastically deformable contact sections, such that they will conform a little to the surface of the object (6) giving a more 'soft' contact to the object, thus helping to prevent it from being damaged, and in addition giving an even further tightened grab of the object. Again the surface of these contact surfaces may be such that they have a high friction.

In one embodiment at least a significant part of the contact surface of the grippers (2) to the object (6) is made of or at least comprises a high friction material of any kind as known in the art.

The coupling (18) may be such that the grippers (2) are detachable from the driving unit (17), and the driving unit (17) may comprise any possible means making it connectable to and detachable from the mounting means (9).

The grippers (2) may automatically sense when they touch the object (6), for example due to the increased resistance to the drivers of the grippers (2). The system then optionally is deactivated and the grip released when the user deactivates the activation switches (5), or alternatively when the user activates a deactivation switch (not illustrated) that may be positioned anywhere and may optionally be to be activated by the other hand than whereto the aid device (1) is attached.

Alternatively the grippers (2) stop e.g. when the fingers (3) release the activation switches (5), and then hold this position until the activation switch (5) is activated a next time.

When the user wants to let the object free, the push of the fingers (3) on the switch(es) is removed, thus removing the lock of the grippers (2).
This only leaves the user to be able to move the arm and open and close the fingers (3), the activation switch(es) (5) may only need a slight touch to be activated, where this may be an adjustable parameter of the aid tool (1), the activation force of the switch(es) (5) especially being adjusted according to the need of the individual user.

The same activation/deactivation of would also apply to the alternative or additional embodiment where the grab is tightened by a slightly further movement of the grippers (2) in the gripping direction.

The aid tool (1) in the same manner as also described in patent application PCT/DK2011/000044 preferably is connected to mounting means (9) for fixing it to the arm of the user, and connecting means (10) connecting the aid tool (1) to the mounting means (9), where the connecting means (10) may comprise joints or other means forming a flexibility of the aid tool (1) in up and down and side to side movements, in the same manner as described in PCT/DK2011/000044, and in the same manner it is preferred that this flexibility is also locked when the grab is activated and a hold established, and unlocked again when the grab and hold is released.

The procedure of operation of the aid tool (1) according to the main embodiment is:
1. The person using the aid tool (1) positions it to form a loose grab on an object (6)
2. The user the tightens the grab until a predetermined force when the activation switch (5) is activated
3. The grippers (2) being locked unidirectional.
4. A hold of the object (6) is being established.

Since the user is to grab the object (6) him- or herself to start with, the aid tool (1) assisting in forming a firm grip, it is essential that the aid tool (1) are equipped with means (8) forming a contact between the aid tool (1) and the hand and/or fingers (3) of the user, where the contact is unidirectional in a manner where it enables the user to grab onto the object, but where the hand/fingers (3) of the user at any time are free to disconnect from the aid tool (1) in the direction of releasing the grab.

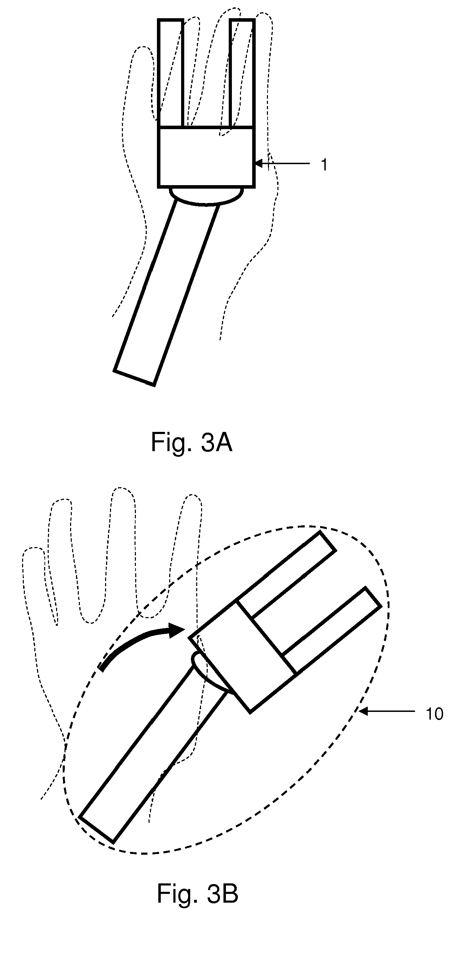

Fig. 3A and 3B shows the aid tool (1) as described above, but where a first embodiment aspect of the present invention is further illustrated. When the user wants to use the grapping tool it is positioned as also described above in the hand such that the user with the hand and fingers may form the first grapping onto an object (6), this is also seen in Fig. 3A.

When the user needs no assistance from the aid tool (1) but neither wants to disconnect it, then it would inhibit the action of the hand when in position as illustrated in e.g. Fig. 3A, and therefore the aid tool (1) comprises means such that it may be pushed aside as illustrated in Fig. 3B.

By 'pushing aside' is meant that the aid tool (1) comprises a movable section (10) that includes the grippers (2) and is connected to the mounting means in a manner where it can be pushed away from the immediate operation range of the hand such as to one of the sides as seen in Fig. 3B.

This could be realized by connecting the movable section (10) to the mounting means through joints allowing such a movement, or through bendable sections. How to realize this would be recognized by person skilled in the art.

When the movable section (10) is pushed aside, then it 'locks' into such a position, simply due to some built in resilience in the movement requiring some force to push it aside, where this resilience then will keep it in the position. This is much as steel wire that with some resilience may be bent, but where it will keep the bending until changed again.

In alternative versions the movable section (10) is drawn back to a position underneath the driving unit (17), either by a rotation thought a joint or simple through a translation of the movable section (10).

Neither the mounting means nor any joints etc. is to be seen in the figure, as the important aspect of the present invention is not how this is realized, a person skilled in the art would know best to do it.

When the movable section (10) is in the pushed aside position, then to regain it the user needs to grab it and take it back to the operating position within the hand and/or fingers. If it is locked into the position aside position as described above, then in one embodiment of the present invention the user needs to activate the unlocking of the movable section (10), e.g. by activating a switch, e.g. the same activation switch (5) as is used to when to establish a hold. Alternatively the unlocking may be done when the user presses the grippers (2) with a force, e.g. a force smaller than the predetermined activation force. This could be measured e.g. by measuring the strain gauge of one or more of the grippers (2), or alternatively by introducing a force sensor into the system, in both cases measuring the actual force by which the grippers (2) grabs onto the object (6), where such measurements may be used to find the predetermined force with which the activation switch (5) is to be pushed to activate the hold. But it may also be utilized in relation to the pushing aside of the movable section (10). In one embodiment this force needs to be maintained for a given time before the unlocking appears.

In an alternative or additional embodiment of the present invention, the movable section automatically moves to the aside position when not in use, e.g. when no activation has been done for a given time.

In one embodiment of the present invention the movable section (10) is movable in a side-to-side movement and an up-and-down movement, thus able to follow the movement of the hand, and therefore it is possible for the user to grab an object at different angles, which has been shown often to be required. This mobility of the naturally is realized through the same means as those making the push aside of the movable section (10) possible. In the same manner and by the same technical means as the movable section (10) may be locked when pushed aside, it may lock into the actual position when the hold is activated.

In the present preferred but not limiting embodiment the movable section (10) is operated through a set of strings. The strings are loosened or tightened by an electromotor winding or unwinding the strings onto a wheel. The locking of the movable section (10) into a position may be realized by the strings being tightened thus e.g. drawing a pin into a clutch in a manner where the position of the movable section (10) is locked into the present position.

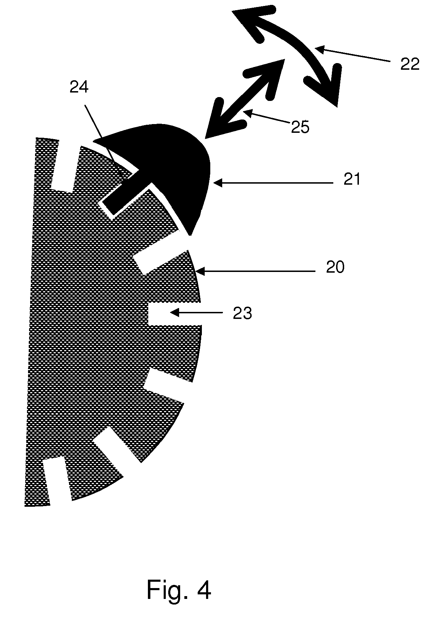

This is illustrated in Fig. 4 where a clutch system (20) is fixed e.g. in connection with or to the mounting means (9) and the pin device (21) is fixed in connection to the movable section (10) being able to rotate (22) sidewise with this relative to the clutch system (20), and to be drawn into (25) one of several holes (23) of the clutch system (20), which of the several holes (23) depending on the relative position of the pin device (21) to the clutch system (20). To guide the pin device (21) into one of the holes (23) if not perfectly aligned, the holes (23) with advantage may have tapered inlets.

The pin device (21) may be operated by strings, or wires, drawing (25) the pin (24) into a hole (23) when the strings are tightened locking for any rotation (22) of the pin device (21), and when released it pulls out again (25) freeing the pin device (21) to rotate again.

The strings of these embodiments may be the wires (12) also connected pulling the grippers (2), and/or may be operated in a similar manner.

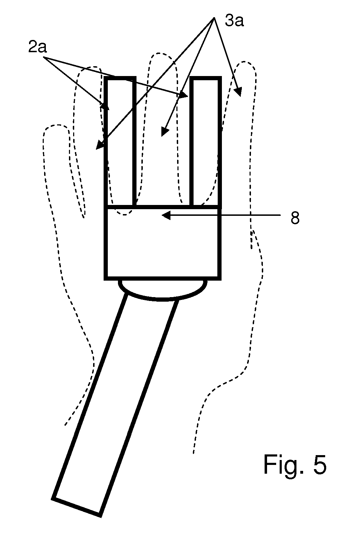

Fig. 5 a further possible additional or alternative embodiment is illustrated to the system of one or all of Figs. 1-3 seen as a top view, where the fingers (3a) are seen being positioned between (and at the side) the grippers (2a), and where the contact platform (8) are illustrated as an embodiment where the contact are formed to the palm of the hand.

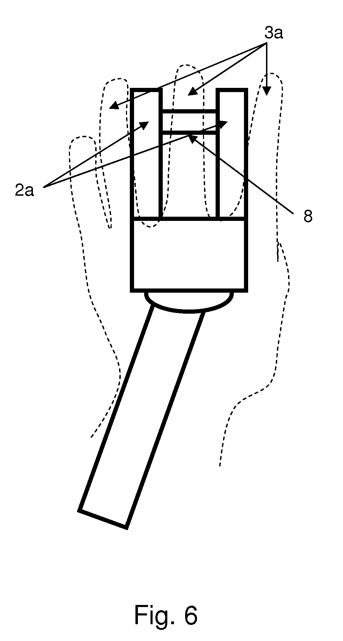

Fig. 6 illustrates and alternative or additional embodiment where there alternatively or additionally is a contact platform (8) positioned such that it forms contact to the upper half of a finger (3a), and may wholly or partly between two neighbouring grippers (2) and may be fixed to one or both of these.

The contact platform (8) may be a rigid bar or strip member, it may be arch shaped in a manner where it is adapted to receive the associated finger, it may be a flexible member extending between two neighbouring grippers, or may be formed in any other convenient manner and of any convenient material

By measuring the strain gauge of one or more of the grippers (2), or alternatively by introducing a force sensor into the system, in both cases measuring the actual force by which the grippers (2) grabs onto the object (6), such measurements may be used to find the predetermined force with which the activation switch (5) is to be pushed to activate the hold.

The user may e.g. be requested at regular intervals to squeeze an object as hard as possible, and then taking this measurement as basis for the new predetermined activation force, either directly or some calculated value based on the measurement.

In the same manner it may be used to exercise the user by increasing the predetermined activation force, thus forcing the user to increase the force they need to apply to form the hold. When the hold is formed, the user no longer needs to use any force to grab the object (6).

The aid device (1) may even be used for exercise programs, where the user are trained by squeezing an object e.g. at different forces and for different time intervals, the activation switch (5) for example only being activated when this 'goal' is achieved.

Such programs may be included in the aid device by equipping it with a computer chip controlling the activation switch (5) and being feed with input from the measured forces. The same computer chip may in addition, or alternatively, be used as a data collecting device, such as a computer chip, and/or may be in data communication with a receiver either to send the measurements, and/or to receive new information/instructions.

The grippers (2a) and (2b) may be connected at a joint being movable independently to each other, or may be connected e.g. by cogwheels or any other manner, such that one naturally will follow the movement of the other, such that only one of them needs to be moved.

## Claims

1. Aid tool (1) to be used in combination with the hand of a person, the aid tool (1) comprising at least two grippers (2) where the grippers (2) are adapted to move at least in the directions to grab and release an object (6),
the aid tool (1) including a movable section (10), said movable section (10) including the grippers (2), and said movable section (10) having an operation position where the grippers (2) are in connection with the hand and/or fingers of the user, and a pushed aside position where they are not in direct connection with the hand and/or fingers of the user or conflicting with the direct up and down rotation of the hand at the wrist, the movable section (10) being connected to mounting means (9) through joints (11) or a bendable section allowing relative movement of the movable section (10) to the mounting means, **characterized in that** the movable section (10) is constructed to lock in the pushed aside position through build in resilience in the joints (11) or bendable section.

2. Aid tool (1) according to claim 1, wherein the movable section (10) being locked in the pushed aside position unlocks again when a switch is activated.

3. Aid tool (1) according to claim 1 or 2, wherein the movable section (10) being locked in the pushed aside position unlocks again when the grippers (2) is pushed or squeezed at some predetermined force.

4. Aid tool (1) according to claim 3, wherein the aid tool (1) includes a force sensor measuring the force whereby the grippers (2) is pushed or squeezed.

5. Aid tool (1) according to claim 3, wherein force whereby the grippers (2) is pushed or squeezed is measured measuring the strain gauge of one or more of the grippers (2).

6. Aid tool according to any of the preceding claims, wherein the movable section (10) automatically moves to the pushed aside position when no activation has been made for a given time.

7. Aid tool according to any of the preceding claims, wherein at least one gripper (2) are adapted to positioned at the side of a finger (3) of the user, defined as being essentially in the orthogonal direction to the direction to grab and release.

8. Aid tool (1) according to claim 7, wherein each of the grippers (2) are positioned at the side of a finger (3).

9. Aid tool (1) according to claim 7 or 8, wherein aid tool (1) comprises a contact platform (8) adapted to contact hand and/or fingers (3) in a manner where this contact platform (8) will be positioned between the hand and/or finger and an object (6) when grapping the object (6).

10. Aid tool (1) according to any of the preceding claims, wherein a contact platform (8) is positioned to contact the palm of the hand.

11. Aid tool (1) according to any of the preceding claims, where an activation of the aid tool (1) locking the grippers (2) unidirectionally such that they are locked in their movement in the releasing direction.

12. Aid tool (1) according to any of the preceding claims, wherein it further comprises means to establish a hold of the object (6), being formed by grippers forming an additional holding force to the object (6) to the one at the time of activation.

13. Aid tool (1) according to claim 8, where the hold is established by the grippers (2) after activation moving in the grapping direction to form a slightly tighter grab than at the time of activation.

## Patentansprüche

1. Hilfswerkzeug (1), das in Kombination mit der Hand einer Person zu benutzen ist, wobei das Hilfswerkzeug (1) mindestens zwei Greifer (2) umfasst, wobei die Greifer (2) dafür ausgelegt sind, um sich mindestens in die Richtungen zu bewegen, um ein Objekt (6) zu greifen und loszulassen,
wobei das Hilfswerkzeug (1) einen beweglichen Abschnitt (10) enthält, wobei der bewegliche Abschnitt (10) die Greifer (2) enthält und wobei der bewegliche Abschnitt (10) eine Betriebsposition aufweist, in der die Greifer (2) in Verbindung mit der Hand und/oder den Fingern des Anwenders stehen, und eine zur Seite geschobene Position aufweist, in der die Greifer nicht in einer direkten Verbindung mit der Hand und/oder den Fingern des Anwenders stehen oder in einem Konflikt mit der direkten Drehung der Hand an dem Handgelenk nach oben und unten stehen, wobei der bewegliche Abschnitt (10) mit den Befestigungsmitteln (9) durch Gelenke (11) oder durch einen biegbaren Abschnitt verbunden ist, der eine relative Bewegung des beweglichen Abschnitts (10) zu den Befestigungsmitteln ermöglicht,
**dadurch gekennzeichnet, dass** der bewegliche Abschnitt (10) konstruiert ist, um in der zur Seite geschobenen Position durch eine eingebaute Ausfallsicherheit in den Gelenken (11) oder in dem biegbaren Abschnitt zu verriegeln.

2. Hilfswerkzeug (1) nach Anspruch 1, wobei der bewegliche Abschnitt (10), der in der zur Seite geschobenen Position verriegelt ist, wieder entriegelt, wenn ein Schalter aktiviert wird.

3. Hilfswerkzeug (1) nach Anspruch 1 oder 2, wobei der bewegliche Abschnitt (10), der in der zur Seite geschobenen Position verriegelt ist, wieder entriegelt, wenn die Greifer (2) mit einer gewissen, vorgegebenen Kraft gestoßen oder gedrückt werden.

4. Hilfswerkzeug (1) nach Anspruch 3, wobei das Hilfswerkzeug (1) einen Kraftsensor enthält, der die Kraft misst, mit der die Greifer (2) gestoßen oder gedrückt werden.

5. Hilfswerkzeug (1) nach Anspruch 3, wobei die Kraft, mit der die Greifer (2) gestoßen oder gedrückt werden, gemessen wird, indem der Dehnungsmessstreifen von einem oder mehreren der Greifer (2) gemessen wird.

6. Hilfswerkzeug nach einem der vorhergehenden Ansprüche, wobei sich der bewegliche Abschnitt (10) automatisch zu der zur Seite geschobenen Position bewegt, wenn für eine gegebene Zeit keine Aktivierung durchgeführt worden ist.

7. Hilfswerkzeug nach einem der vorhergehenden Ansprüche, wobei mindestens ein Greifer (2) dafür ausgelegt ist, an der Seite eines Fingers (3) des Anwenders positioniert zu werden, und der im Wesentlichen in der Richtung, die zu der Richtung zu greifen und loszulassen senkrecht ist, definiert ist.

8. Hilfswerkzeug (1) nach Anspruch 7, wobei jeder der Greifer (2) an der Seite eines Fingers (3) positioniert ist.

9. Hilfswerkzeug (1) nach Anspruch 7 oder 8, wobei das Hilfswerkzeug (1) eine Kontaktplattform (8) umfasst, die dafür ausgelegt ist, um eine Hand und/oder Finger (3) in einer Weise zu berühren, in der diese Kontaktplattform (8) zwischen der Hand und/oder dem Finger und einem Objekt (6) positioniert werden wird, wenn das Objekt (6) ergriffen wird.

10. Hilfswerkzeug (1) nach einem der vorhergehenden Ansprüche, wobei eine Kontaktplattform (8) positioniert ist, um die Handfläche zu berühren.

11. Hilfswerkzeug (1) nach einem der vorhergehenden Ansprüche, wobei eine Aktivierung des Hilfswerkzeugs (1) die Greifer (2) in einer Richtung derart verriegelt, dass sie in ihrer Bewegung in der Richtung des Loslassens verriegelt sind.

12. Hilfswerkzeug (1) nach einem der vorhergehenden Ansprüche, wobei es ferner Mittel umfasst, um einen Halt des Objekts (6) zu errichten, der durch Greifer gebildet wird, die eine zusätzliche Haltekraft auf das Objekt (6) bilden, eine Kraft zusätzlich zu der einen zum Zeitpunkt der Aktivierung.

13. Hilfswerkzeug (1) nach Anspruch 8, wobei der Halt durch die Greifer (2) nach einer Aktivierung einer Bewegung in die Greifrichtung errichtet wird, um einen leicht festeren Griff als zu dem Zeitpunkt der Aktivierung zu bilden.

## Revendications

1. Outil d'aide (1) à utiliser en combinaison avec la main d'une personne, l'outil d'aide (1) comprenant au moins deux organes de préhension (2) où les organes de préhension (2) sont adaptés afin de se déplacer au moins dans les direction pour saisir et lâcher un objet (6), l'outil d'aide (1) incluant une section mobile (10), ladite section mobile (10) incluant les organes de préhension (2) et ladite section mobile (10) ayant une position de fonctionnement, où les organes de préhension (2) sont raccordés à la main et/ou aux doigts de l'utilisateur, et une position poussée de côté où ils ne sont pas raccordés directement à la main et/ou aux doigts de l'utilisateur ou entravant la rotation directe vers le haut et le bas de la main au niveau du poignet, la section mobile (10) étant raccordée au moyen de montage (9) par l'intermédiaire de joints (11) ou une section flexible permettant un mouvement relatif de la section mobile (10) par apport au moyen de montage, **caractérisé en ce que** la section mobile (10) est construite afin de se verrouiller dans la position poussée sur le côté par un accroissement de résilience dans les joints (11) ou la section flexible.

2. Outil d'aide (1) selon la revendication 1, dans lequel la section mobile (10) étant verrouillée dans la position poussée sur le côté se déverrouille en sens contraire quand un interrupteur est activé.

3. Outil d'aide (1) selon la revendication 1 ou 2, dans lequel la section mobile (10) étant verrouillée dans la position poussée sur le côté se déverrouille en sens contraire quand les organes de préhension (2) sont poussés ou pressés à une certaine force prédéterminée.

4. Outil d'aide (1) selon la revendication 3, dans lequel l'outil d'aide (1) inclut un capteur de force mesurant la force moyennant quoi les organes de préhension (2) sont poussés ou pressés.

5. Outil d'aide (1) selon la revendication 3, dans lequel la force par laquelle les organes de préhension (2) sont poussés ou pressés est mesurée en mesurant la jauge de contrainte d'un ou plusieurs des organes de préhension (2).

6. Outil d'aide selon une quelconque des revendications précédentes, dans lequel la section mobile (10) se déplace automatiquement en position poussée sur le côté quand aucune activation n'a été faite pendant un temps donné.

7. Outil d'aide selon une quelconque des revendications précédentes, dans lequel au moins un organe de préhension (2) est adapté pour être positionné sur le côté d'un doigt (3) de l'utilisateur, défini comme étant essentiellement dans la direction orthogonale à la direction pour saisir et lâcher.

8. Outil d'aide (1) selon la revendication 7, dans lequel chacun des organes de préhension (2) est positionné sur le côté d'un doigt (3).

9. Outil d'aide (1) selon la revendication 7 ou 8, dans lequel un outil d' aide (1) comprend une plateforme de contact (8) adaptée pour contacter la main et/ou les doigts (3) d'une manière où cette plateforme de contact (8) sera positionnée entre la main et/ou le doigt et un objet (6) lorsqu'un objet (6) est saisi.

10. Outil d'aide (1) selon une quelconque des revendications précédentes, dans lequel une plateforme de contact (8) est positionnée afin de contacter la paume de la main.

11. Outil d'aide (1) selon une quelconque des revendications précédentes, dans lequel une activation de l'outil d'aide (1) verrouille les organes de préhension (2) unidirectionnellement de sorte qu'ils soient verrouillés dans leur mouvement dans la direction pour lâcher.

12. Outil d'aide (1) selon une quelconque des revendications précédentes, dans lequel il comprend en outre un moyen pour établir une retenue de l'objet (6), étant formé par les organes de préhension formant une force de retenue additionnelle de l'objet (6) au moment de l'activation.

13. Outil d'aide (1) selon la revendication 8, dans lequel la retenue est établie par les organes de préhension (2) après l'activation se déplaçant dans la direction de saisie afin de former une saisie légèrement plus ferme au moment de l'activation.
